(19) Europäisches Patentamt

European Patent Office

Office européen des brevets

(11) **EP 0 503 824 B1**

(12) **EUROPEAN PATENT SPECIFICATION**

(45) Date of publication and mention
of the grant of the patent:
**27.08.1997 Bulletin 1997/35**

(51) Int. Cl.$^6$: **A61L 25/00**, C08J 9/02,
B65D 81/32, B65D 83/14,
D65D 83/76

(21) Application number: **92301831.1**

(22) Date of filing: **04.03.1992**

(54) **Apparatus for mixing and dispensing a multicomponent composition**

Vorrichtung zum Mengen und Abgeben von einer Mehrkomponentenzusammensetzung

Dispositif mélangeur et distributeur pour une composition à plusieurs composants

(84) Designated Contracting States:
**DE FR GB IT**

(30) Priority: **13.03.1991 FR 9103016**

(43) Date of publication of application:
**16.09.1992 Bulletin 1992/38**

(73) Proprietor: **Dow Corning France S.A.**
**F-06561 Valbonne (FR)**

(72) Inventors:
• **Bouquet, Philippe Louis**
**F-06370 Mouans-Sartoux (FR)**
• **Pocknell, David**
**Rhoose, South Glamorgan, Wales (GB)**

• **Bruning, Werner**
**W-5250 Engelskirchen (DE)**
• **Dittrich-Duster, Martina**
**W-5250 Engelskirchen (DE)**
• **Lamers, Gunter**
**W-5250 Engelskirchen (DE)**

(74) Representative: **Bullows, Michael**
**Dow Corning Limited,**
**Cardiff Road**
**Barry, South Glamorgan CF63 7YL, Wales (GB)**

(56) References cited:
EP-A- 0 124 852      EP-A- 0 223 409
EP-A- 0 330 723      FR-A- 2 412 475
FR-A- 2 589 737      FR-A- 2 589 832

Printed by Rank Xerox (UK) Business Services
2.14.12/3.4

## Description

This invention is concerned with improvements in or relating to apparatus for mixing and dispensing a multi-component composition, for example a two part room temperature curable silicone foam.

Liquid curable compositions are available which flow and foam readily at room or slightly elevated temperature to provide a cured foam product. It has been proposed to employ foamable silicone based room temperature curable compositions for various purposes, including the preparation of medical dressings. Compositions for this purpose are disclosed, for example, in French Patent Specification 2589872. The compositions referred to therein comprise an organosilicon polymer including siloxane units providing a silicon-bonded hydroxyl group, an organosilicon polymer including siloxane units having a silicon-bonded hydrogen atom, a catalyst, for example a tin compound, and finely divided filler comprising silica which has been treated to render it hydrophobic. The compositions cure according to the scheme $\equiv$SiOH + $\equiv$SiH --> $\equiv$Si-O-Si$\equiv$ + H$_2$.

Whilst satisfactory in many ways, the tin catalysed compositions disclosed in French Patent Specification 2589872 are regarded as less than satisfactory in that it has been suggested that the tin compound catalysts and/or derivatives thereof may have some undesirable toxic effects.

Formulations have been proposed for silicone rubber foams which do not use tin compound catalysts. Many of these formulations employ polydiorganosiloxanes having silicon-bonded vinyl groups available for reaction with polydiorganosiloxanes having silicon bonded hydrogen atoms and a platinum catalyst. The addition reaction which occurs is appropriate to yield chain extended or cross-linked elastomeric silicone products, but does not generate volatile materials for causing foaming in the curing composition. A foaming reaction may be induced in such formulations by inclusion of a polydiorganosiloxane having silicon-bonded hydroxyl groups among the ingredients with a view to reaction with the polydiorganosiloxane having silicon-bonded hydrogen atoms as more fully described for example in U.S. 4,026,845, with or without the presence of water or an aliphatic alcohol as more fully described for example in U.S. 4,613,630, or by inclusion in the composition of a volatile blowing agent as more fully described for example in U.S. 4,550,125. Hitherto, our attempts to employ foamable compositions based on polydiorganosiloxanes having silicon bonded hydrogen atoms and silicon-bonded vinyl groups have not resulted in compositions which cure and foam suitably to form foamed dressings in situ on a patient's body. In particular they do not always cure satisfactorily in contact with wet wound surfaces and may even exhibit an uncured, liquid surface layer; some cure too slowly for convenient use and others do not yield a foam of desirably low density and structure for convenient use as an in situ formed medical dressing. Desirably, compositions intended for in situ provision of medical dressings are curable at room temperatures of the order of 20°C ± 4°C within 100 seconds ± 40 seconds of application to the body to yield a foam of uniform fine pores having a density between 100 Kg/m$^3$ and 400 Kg/m$^3$ and having a major proportion of open cells.

We have now found that a foam forming composition suitable for use in preparation of in situ formed dressings may comprise two components having similar viscosities each of which may be stored in a receptacle designed to be ruptured to release the components for admixture. Suitable foam forming compositions include those comprising one or more polydiorganosiloxanes having not less than three alkyl-hydrogensiloxane units per molecule, (B) one or more polydiorganosiloxanes having not less than two siloxane units of the formula

$$R_a R'_b SiO_{\frac{(4-(a+b))}{2}}$$

in which R represents a monovalent hydrocarbon group containing 1 to 20 carbon atoms, R' represents an unsaturated hydrocarbon group, $a$ has the value 0, 1 or 2 and $b$ has the value 1 or 2, (C) a liquid alcohol, (D) a fluorinated silicone foam stabilizing material and (E) a platinum catalyst for promoting reaction between the components.

It is an object of this invention to provide an improved hand held device suitable for the storage, mixing and dispensing of silicone compositions whether foamable or not, especially those which are packaged as separate components of substantially equal viscosities and in substantially equal volumes.

The present invention provides in one of its aspects a mixing and dispensing device adapted to be held in one hand of an operator for mixing together the components of a silicone composition packaged in the form of two or more individually stable components of substantially equal volume and viscosity each in a receptacle, and to dispense the mixed composition by a manual operation, the device comprising a mixing housing in which there is a mixing region into which the components of the multi-component composition may be introduced and mixed together, separate chambers each comprising one of said receptacles or forming a receptacle having a closure made of a material which can be ruptured, each chamber communicating with the mixing region, piercing means which is arranged so that when it is desired to release a component from its receptacle the piercing means may be caused to pierce the receptacle, plunger means for expelling a component from the chamber by forcing it into the mixing region, a mixing element located in the mixing region for mixing the components of the composition, and a passageway which contains means for actuating the mixing element and through which the mixed composition may be expelled from the mixing region, the construction and arrangement being such that when it is desired to mix and dispense the composition relative movement of approach may be caused between the piercing means and the receptacles to bring about rupture of the receptacles, relative movement of approach may be brought about between the plunger means and the mixing housing to urge the component into the mixing region, the mixing element may be actuated to mix the components in the mixing region, and relative movement of approach may be brought about between elements of the device to discharge the mixed composition from the device through said passageway.

Two devices according to the invention are hereinafter described to illustrate the invention. These illustrative devices are adapted to be held in one hand of an operator for mixing together the components of a multi-component composition and for dispensing the mixed composition by manual operation. Each illustrative device has a mixing housing in which there is a mixing region and chambers for housing the parts of the composition. In the illustrative devices the chamber support comprises two cylindrical chambers, the axes of which are parallel and located at either side of, and parallel to, a centre line of the chamber support. The chambers are located at approximately 180° about the axis of the mixing housing. Such embodiments are useful for the mixing and dispensing of two component compositions. The parallel, that is side-by-side, location of the two chambers facilitates loading of the components into the chambers particularly when discrete envelopes of components are used.

In a device according to the invention, the components are expelled from their chambers into the mixing region by action of the plunger means. Preferably, the plunger means is arranged so that a single actuation of the plunger means causes delivery of the desired proportions of the components from the chambers simultaneously. The illustrative devices have a plunger for each chamber and two plungers which are rigidly connected together and which are mounted for simultaneous sliding movement within the chambers.

A device according to the invention has piercing means which may be caused to penetrate a container present in the chamber. Although the means for piercing the container may be formed so as to completely cut away part of the container, for example by cutting a circular shape, it is preferred that the means for piercing the container is arranged so that the part of the container which is cut remains attached to the container and so does not pass into the mixing housing with the components of the composition. The means for piercing the container may for example, cut a part of, but not a complete, circular shape. The means for piercing may comprise a needle or blade. In the first illustrative device, the means for piercing the envelope is a circular or part elliptical knife blade and is formed integrally with the chamber support. Ports extend through the centre of the piercing means and communicate between the chambers and the mixing region. In the second illustrative device the means for piercing the envelope is provided by knife means supported on the mixing element.

A device according to the invention is provided with means for rotating, reciprocating or otherwise agitating the mixing element to effect mixing of the combined components. In the illustrative devices the mixing element is releasably secured to a mixer rod which extends through the device and has a handle by means of which the mixing element may be moved reciprocally in the mixing region for mixing the components in the mixing region. The mixer rod is releasably secured to the mixing element by a screw-thread connection. The mixing element is preferably restrained from rotation in the mixing housing about the axis of the mixing housing so that the mixer rod can be removed from the device after mixing has been accomplished, for example by provision of splines or abutments or by arranging that the relative shapes and sizes of the mixing housing and the mixing element are such as to restrain such rotation. In the first illustrative device, the mixing housing and the mixing element are oval in shape and the narrowest cross-sectional width of the mixing housing is narrower than the widest cross-sectional width of the mixing element so that the mixing element cannot fully rotate in the mixing housing. In the second illustrative device splines are used for the same purpose.

A device according to the invention is preferably sealed against ingress of foreign matter.

The first illustrative device comprises

a) a mixing housing comprising a base having an interior surface and a wall having an interior wall surface;

b) a chamber support providing parallel chambers, one for each component of the multi-component composition, the chamber support having a base and a wall, each having an exterior surface, the chamber support being slidable in the housing and the exterior surface of the base of the chamber support and the interior surfaces of the housing base and housing wall being such that together they define a mixing region into which the components of a multi-component composition may be introduced and mixed together, each chamber comprising a wall extending from an interior surface of the base of the chamber support and the chamber wall having an interior surface and each chamber communicating with the mixing region via a port in the base of the chamber support;

c) piercing means which is arranged so that when it is desired to release a component from a receptacle located in the chamber the piercing means may be caused to pierce the receptacle;

d) plunger means slidably engaged with the interior wall surface of each chamber suitable for expelling a component from the chamber into the mixing region;

e) a perforate mixing element located in the mixing region;

f) means for causing movement of the mixing element in the mixing region for mixing the components of the composition, and

g) a passageway located between the chambers and communicating with the mixing region at one end and providing a nozzle at its other end so that the mixed composition may be expelled from the mixing region via the passage-way upon bringing about relative movement of approach between the mixing housing and the chamber support.

The second illustrative device comprises a mixing and dispensing device adapted to be held in one hand of an operator for mixing together the components of a multicomponent composition and for dispensing the mixed composition by manual operation comprising

a) a mixing housing comprising a base having an interior surface and a wall having an interior wall surface;

b) a chamber support having parallel chambers, one for each component of the multi-component composition, the chamber support being fixed to the wall of the mixing housing, such that together they define a mixing region into which the components of a multi-component composition may be introduced and mixed together, each chamber communicating with the mixing region via a port in the chamber support;

c) a perforate mixing element located in the mixing region;

d) piercing means which is arranged so that when it is desired to release a component from a chamber the piercing means may be caused to pierce a retaining surface to release the component from the chamber;

e) plunger means slidably engaged with the interior wall surface of each chamber suitable for expelling a component from the chamber into the mixing region;

f) a passageway communicating with the mixing region at one end and providing a nozzle at its other end so that the mixed composition may be expelled from the mixing region via the passageway upon bringing about relative movement of approach between the mixing housing and the chamber support, and

g) means for causing movement of the mixing element in the mixing region for mixing the components of the composition.

A device according to the invention is particularly suitable for use for mixing and dispensing sterile components of a composition which may be supplied in sterile discrete receptacles. Thus, for example, the device may be used for the sterile dispensing of compositions intended for medical use, for example as medical dressings on, or for use on, the human or animal body.

There now follows a detailed description, to be read with the accompanying drawings, of the illustrative devices and a modified form of the first illustrative device.

In the drawings:

Figure 1 is a side elevation of a receptacle containing one component part of a composition;
Figure 2 is a section of the receptacle shown in Figure 1;
Figure 3 is a section of the first illustrative device;
Figure 4 is a section taken substantially on the line X - X of Figure 3;
Figure 5 is a section taken substantially on the line Y - Y of Figure 3;
Figure 6 is a section similar to Figure 3 but showing a portion of the first illustrative device during operation of the device;
Figure 7 is a section of a chamber of the modified form of the first illustrative device containing an intact receptacle in the form of a bellows;
Figure 8 is a section taken substantially on the line Z - Z of Figure 7;
Figure 9 is a section similar to Figure 7 but showing the receptacle during operation of the device, and
Figure 10 is a sectional view of the second illustrative device.

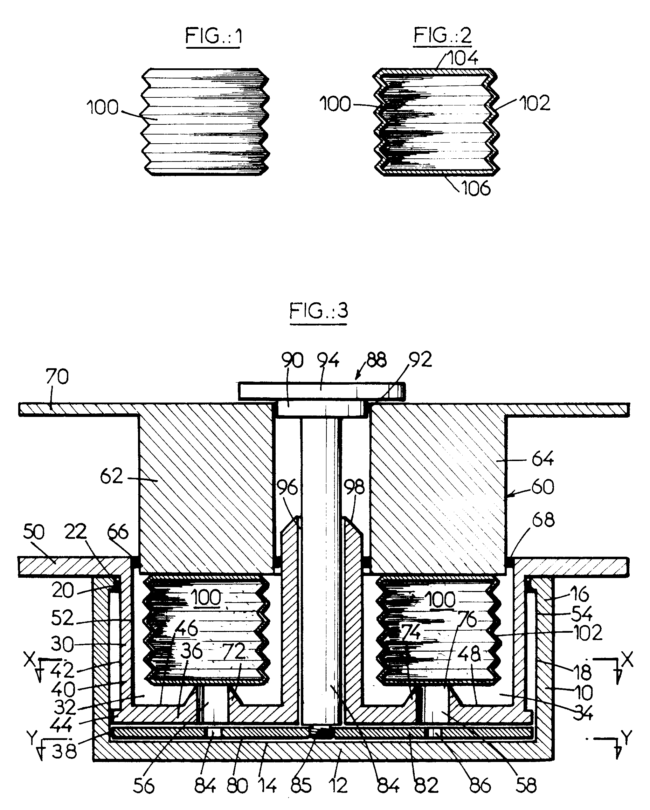

An example composition was prepared comprising two components of substantially equal viscosity and volume of a silicone composition capable of cure at room temperature to provide a fine pored foam. For use in the first illustrative device, the component parts A and B were packaged separately in receptacles having the shape of a cylindrical bellows (100 Figures 1 and 2). Each bellows was formed from polypropylene and comprises corrugated walls (102) and reinforced upper (104) and lower (106) surfaces. The construction and arrangement of each receptacle is such that when the receptacle is perforated to release the component, a comparatively light pressure is sufficient to cause controlled collapse of the receptacle in the direction of its axis so that the bellows may be emptied.

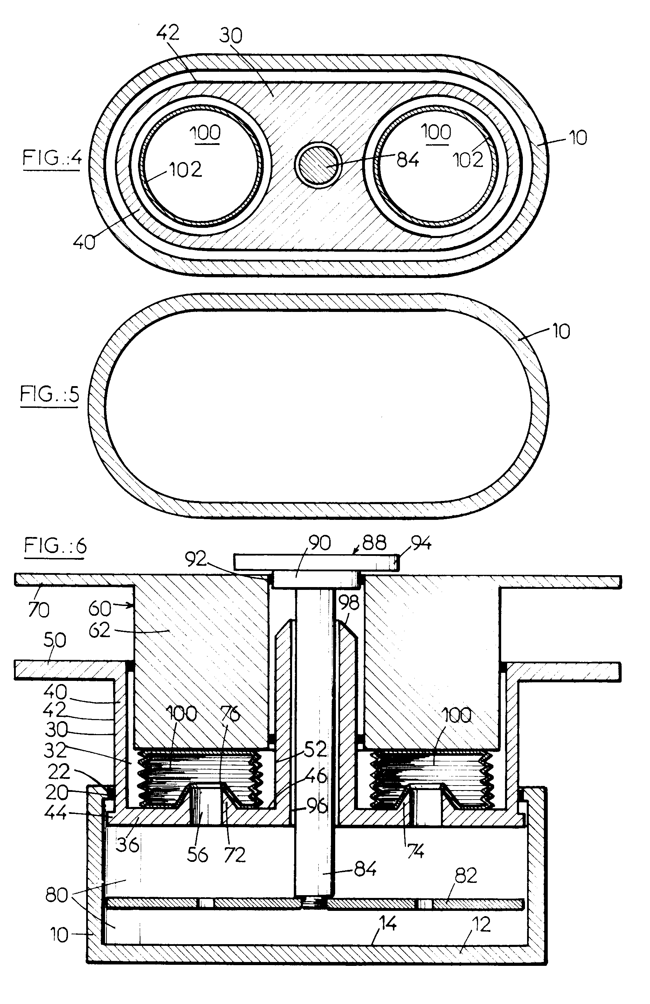

The first illustrative device comprises a mixing housing (10) (Figures 3, 4 and 5) which has a generally oval configuration, having parallel sides and rounded ends. The mixing housing (10) comprises a base (12) having a continuous interior base surface (14) (Figure 3) and a wall (16) upstanding fom the base (12) which has an interior wall surface (18). A chamber support (30) is mounted for sliding movement in the mixing housing (10). The chamber support (30) comprises two circular chambers (32 and 34) the axes of which are parallel and located at either side of and parallel to a centre line of the chamber support. The chamber support (30) has a base (36) having an exterior base surface (38) and a wall (40) upstanding from the base (36) which has an exterior wall surface (42). The wall (16) of the mixing housing (10) has an inwardly extending lip (20) at its upper end (as viewed in Figure 3) which extends entirely around the upper edge of the wall (16) and carries a sealing ring (22). The wall (40) of the chamber support (30) has a shape such that the exterior wall surface (42) engages firmly with the sealing ring (22) so that a seal is provided between the walls of the chamber support and the mixing housing. An abutment (44) extends around the chamber support (30) at its base and serves to stabilise the chamber support in the mixing housing and to co-operate with the lip (20) to prevent the mixing housing and chamber support from becoming separated inadvertently. The exterior surface (38) of the base of the chamber support (30), the interior surface (14) of the base (12) of the mixing housing (10) and the interior surface (18) of the wall (16) of the mixing housing together define a mixing region (80).

The device has plunger means (60) comprising two plungers (62, 64) which are rigidly connected together and which are mounted for simultaneous sliding movement within the chambers (32, 34 respectively). Between each chamber (32, 34) and its plunger (62, 64) there is a sealing ring (66, 68). Each chamber (32, 34) is circular in section and is defined by an interior surface (46, 48) of the base (36) of the chamber support, a generally cylindrical interior wall surface (52, 54) in the chamber support (30) and a surface of its plunger. The chambers (32) and (34) communicate with the mixing region (80) via ports (56, 58) in the base of the chamber support (30). The extent to which each plunger (62, 64) may penetrate its chamber is limited by flanges (50) on the chamber support (30) and flanges (70) on the plunger means (60). These flanges are useful for handling the device and for moving its parts relative to one another. The chambers (32, 34) are shaped to receive a container (100, 102). The interior base surface (46, 48) of each chamber has piercing means provided by an upstanding conical piercing element (72, 74) formed integrally with the chamber support (30). The ports (56, 58) extend through the piercing elements (72, 74 respectively) centrally so that the piercing elements are provided with a circular cutting edge (76) disposed normal to the axis of its chamber.

A perforate mixing element (82) is contained within the mixing region (80) and has holes (84, 86) therein. The mixing element (82) is releasably secured to a mixer rod (84) by a screw-thread connection (85). The mixer rod (84) has a protuberance (88) at the end opposite to that to which the mixing element (82) is connected. A lower (as viewed in Figure 3) portion (90) of the protuberance (88) closes an aperture in the plunger means (60) between the plungers (62, 64). A sealing ring (92) is located between the lower portion (90) and the plunger means. An upper (as viewed in Figure 3) portion (94) of the protuberance (88) serves as a handle for the mixer rod by means of which the mixing element may be moved reciprocally in the mixing region and by means of which the mixer rod may be unscrewed from the mixing element.

The mixer rod (84) passes through a passageway (96) formed in the chamber support (30). The passageway (96) is located substantially centrally between the chambers. One end of the passageway (96) communicates with the mixing region (80) and its opposite end provides a nozzle (98).

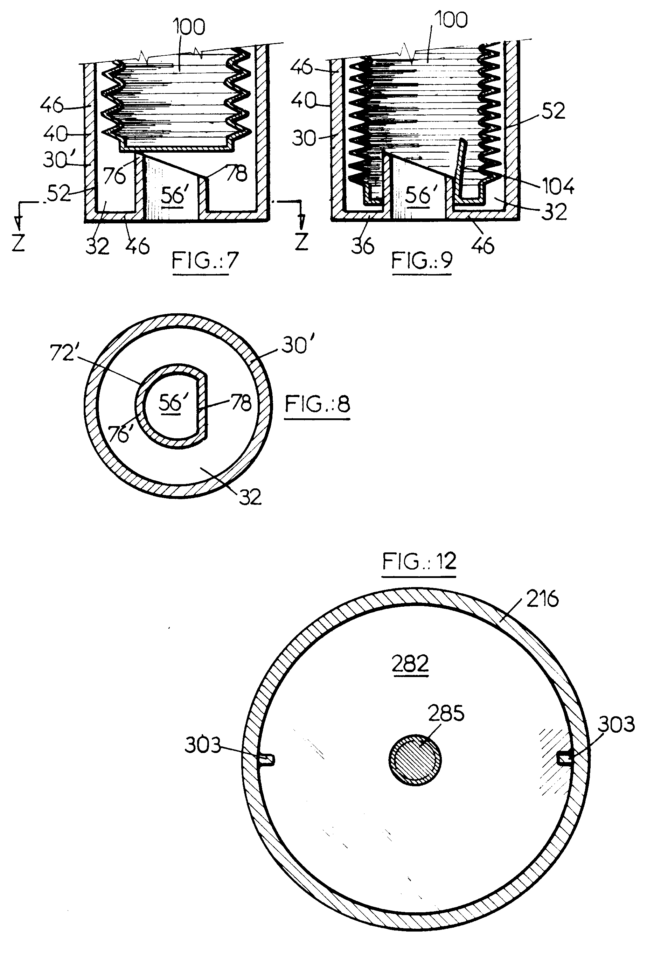

The modified form of the first illustrative device is the same as the first illustrative device except that the piercing means is of a different design. In the modified form (Figures 7, 8 and 9) the piercing means is provided by an upstanding piercing element in the form of a rib (72′, 74′ not shown ) formed integrally with the chamber support (30′) and which describes a closed figure through which the port (56′ and 58′ not shown ) extends. Each piercing element (72′, 74′) comprises a part elliptical cutting edge (76′) which is inclined to the axis of its chamber, and a straight, blunt edge (78).

Operation of the first illustrative device, using the two components of the two component room temperature curable composition which are received in two separate receptacles (100, 102) will now be described. When assembled with the receptacles in the chambers (32, 34) and prior to use, the parts of the device occupy the positions shown in Figure 3. In this condition, the mixing element (82) is trapped between the mixing housing (10) and the chamber support (30) with nothing present in the mixing region other than the mixing element. The plungers (62, 64) rest upon the receptacles and exert no compressive force on them. The mixer rod (84) is in threaded connection with the mixing element and the lower portion (90) of the protuberance closes the plunger means against ingress of air and foreign matter. When it is desired to operate the device, it may be held in one hand and the flanges (50 and 70) drawn towards each other whereby the plungers (62, 64) are caused to penetrate the chambers to compress the receptacles, and the mixer rod (94) is pushed through the chamber support (30). As the receptacles are compressed in the chambers, they are pierced by the piercing elements (72 and 74). Further penetration of the plungers into the chambers causes the expulsion of the contents of the receptacles (100, 102) into the mixing region (50). Figure 6 shows the device when most of the contents of the receptacles (100, 102) have been expelled into the mixing region. When expulsion of the components into the mixing region has been completed, the mixer rod (84) is reciprocated for about 15 seconds to cause the mixing element to move in the mixing region for mixing the components of the composition in the mixing region. The mixer rod (84) is disconnected by unscrewing from the mixing element (82) and removed from the device. The mixed contents of the mixing housing (50) are expelled from the device by holding the flanges (50 and 70) and pushing the mixing housing (10) toward the chamber support (30) so that the mixed contents from the mixing region (80) are urged to pass along the passageway (96) and through the nozzle (98).

When using the modified form of the first illustrative device (Figures 5, 6 and 7) when the receptacles (100, 102) are pushed onto the means for piercing the receptacle, the partially elliptical cutting edge (76′) cuts the receptacle but the part of the receptacle which abuts against the straight, blunt edge (78) is not cut and so a flap (104) is formed. In this way the possibility is avoided that a disc cut from the receptacle may contaminate the composition.

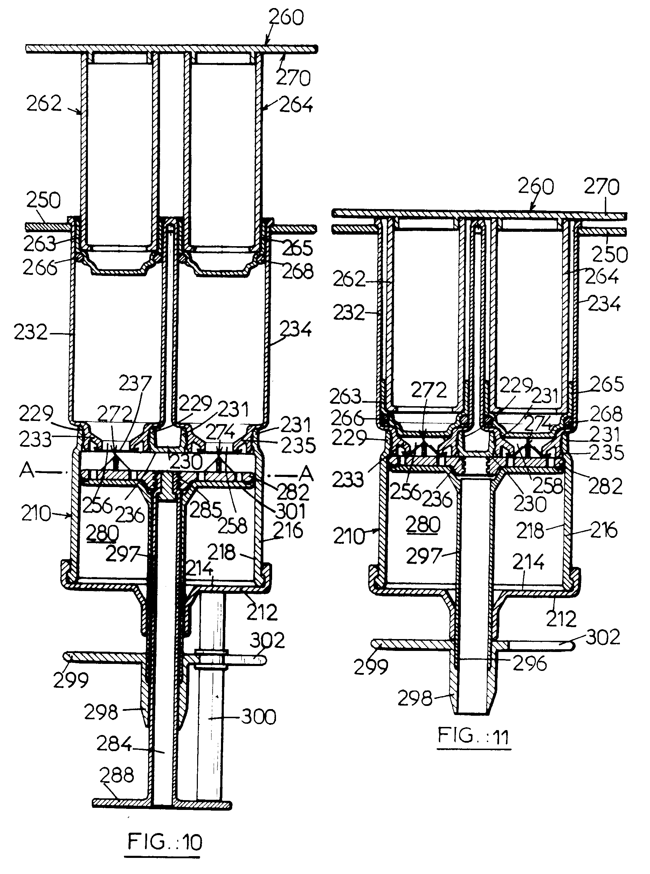

The second illustrative device (Figs 10-12) comprises a mixing housing (210) which has a generally circular configuration and comprises a base (212) having an interior base surface (214) and which is fixed to a cylindrical wall (216) upstanding from the base (212) which has an interior wall surface (218). An upper end (as viewed in Figure 10) portion of the mixing housing is closed by a circular wall which provides a chamber support (230). The chamber support is formed with circular openings (256, 258) each of which is bounded by an upstanding flexible locating lug in the form of a ring (229, 231). Each ring is adapted to receive a neck (233, 235) of a generally cylindrical receptacle or chamber (232, 234) so that the axes of the chambers are parallel and located at either side of and parallel to a centre line of the chamber support (230). Each neck is provided with clip means which co-operate with the rings (229, 231) to secure the chambers (232, 234) to the support (230). The chamber support (230) has an exterior base surface (236) disposed towards the mixing housing. The base surface (236) of the chamber support (230), the interior surface (214) of the mixing housing (210) and the interior surface (218) of the wall (216) of the mixing housing together define a mixing region (280). The base (230) is formed to provide ports (256, 258) communicating between the chambers and the mixing region.

The chambers (232, 234) each contain one of the component parts of the illustrative composition. They are sealed by piston elements (263, 265) configured to mate with the interior of the neck (233, 235) of the chamber and sealed at their lower ends by an aluminium foil (not shown) stretched across an opening in the neck. During storage the piston elements are located at the top (as viewed in Figure 10) of the chambers with composition sealed in the chambers between the piston elements and the aluminium foil. Sealing rings (266, 268) are located between the piston elements and the interior walls of the chambers. Upper end portions of the chambers are received in a flange element (250) adapted to be gripped by the fingers of an operator.

The device has plunger means (260) comprising two plungers (262, 264) which are rigidly connected together and which are mounted in the piston elements (263, 265) and arranged for simultaneous advancement towards the mixing housing to urge the piston elements axially into the chambers (232, 234) towards the necks thereof simultaneously.

The chambers (232, 234) are located to communicate with the mixing region (280, not shown) via the ports (256, 258) in the base of the chamber support (230). The extent to which each plunger (262, 264) may penetrate its chamber is limited by the piston elements and the necks of the chambers and by the flange (250) on the chamber support (230) and flanges (270) on the plunger means (260). These flanges are useful for handling the device and for moving its parts relative to one another.

A perforate mixing element (282) is contained within the mixing region (280) and is releasably secured to a mixer rod (284) by a screw-thread connection (285). Piercing means in the form of upstanding conical piercing elements (272, 274) are provided on the mixing element for perforating the foils (237) blocking the ports (256, 258). The mixer rod (284) is housed in a passageway (296) in a tube (297) which extends through the mixing housing (210) and which carries a piston (301) at its end situated in the housing 210). The tube also carries a nozzle (298) having flanges (299). The passageway (296) is located substantially centrally between the chambers. One end of the passageway (296) communicates with the mixing region (280) and its opposite end extends into the nozzle (298). Outer end portions of the mixer rod are formed with flange portions (288).

Prior to use the mixing element (282) is located between the mixing housing (210) and the chamber support (230) with the piercing elements drawn away from the chambers sufficiently to avoid contact between the piercing elements and the aluminium foils of the chambers. A locking device is provided which comprises a separator rod (300) located parallel to the rod (284) and in contact with the base (212) and a flange (288) and extends through a slot (302) in a flange (299). The rod has lugs thereon which are engaged at either side of the flange (299). The locking device serves to ensure the mixing element is not moved inadvertently relative to the chambers. In order to load the device two chambers (each containing a component part of the silicone composition) are clipped into place on the support (230). The plungers are located in the piston elements. The device is stored with the plungers (262, 264) resting upon the piston elements and exerting no compressive force on them. The mixer rod (284) is in threaded connection with the mixing element and the locking device is in place. When it is desired to operate the device it may be held in one hand and the separator rod (300) of the locking device removed. The rod (284) may be moved to push the mixing element (282) towards the chambers (232, 234) to an extent sufficient for the piercing elements (272, 274) to pierce the foils. Then the flanges (250, 270) may be drawn towards each other whereby the plungers (262, 264) are caused to push the piston elements down the chambers to urge their contents into the mixing region (280). When expulsion of the components into the mixing region has been completed the mixer rod (284) is reciprocated to cause the mixing element to reciprocate in the mixing region to mix the components of the composition in the mixing region. The mixer rod (284) is disconnected by unscrewing from the mixing element (282) and removed from the device. The mixed contents of the mixing housing (210) are expelled from the device by holding the flanges (270, 299) and moving the nozzle (298) and thus the tube (297) and mixing element towards the chamber support (230) so that the mixed contents from the mixing region (280) are urged to pass along the passageway (296) and through the nozzle (298).

**Claims**

1. A mixing and dispensing device adapted to be held in one hand of an operator for mixing together the components of a silicone composition packaged in the form of two or more individually stable components of substantially equal volume and viscosity each in a receptacle (100), and to dispense the mixed composition by a manual operation, the device comprising a mixing housing (10; 210) in which there is a mixing region (80; 280) into which the components of the multi-component composition may be introduced and mixed together, separate chambers (32, 34; 232, 234) each comprising one of said receptacles (100) or forming a receptacle (100) having a closure made of a material which can be ruptured, each chamber (32, 34; 232, 234) communicating with the mixing region (80; 280), piercing means (72, 74; 272, 274) which is arranged so that when it is desired to release a component from its receptacle (100) the piercing means (72, 74; 272, 274) may be caused to pierce the receptacle (100), plunger means (60; 260) for expelling a component from the chamber (32, 34; 232, 234) by forcing it into the mixing region (80; 280), a mixing element (82; 282) located in the mixing region (80; 280) for mixing the components of the composition, and a passageway (96; 296) which contains means for actuating the mixing element (82; 282) and through which the mixed composition may be expelled from the mixing region (80; 280), the construction and arrangement being such that when it is desired to mix and dispense the composition relative movement of approach may be caused between the piercing means (72, 74; 272, 274) and the receptacles (100) to bring about rupture of the receptacles (100), relative movement of approach may be brought about between the plunger means (60; 260) and the mixing housing (10; 210) to urge the component into the mixing region (80; 280), the mixing element (82; 282) may be actuated to mix the components in the mixing region (80; 280), and relative movement of approach may be brought about between elements of the device to discharge the mixed composition from the device through said passageway (96; 296).

2. A device according to Claim 1 having a chamber support (30; 230) which comprises two cylindrical chambers (32, 34; 232, 234) the axes of which are parallel and located at either side of, and parallel to, a centre line of the chamber support and the plunger means (60; 260) comprises two plungers (62, 64; 262, 264) which are rigidly connected together and which are mounted for simultaneous sliding movement within the chambers (32, 34; 232, 234).

3. A device according to Claim 1 comprising,

a) a mixing housing (10) comprising a base (12) having an interior surface (14) and a wall (16) having an interior wall surface (18);
b) a chamber support (30) providing parallel chambers (32, 34), one for each component of a multi-component composition, the chamber support (30) having a base (36) and a wall (40), each having an exterior surface (38, 42), the chamber support (30) being slidable in the housing (10) and the exterior surface (38) of the base (30) of the chamber support and the interior surfaces (14, 18) of the housing base (12) and housing wall (16) being such that together they define a mixing region (80) into which the components of the multi-component composition may be introduced and mixed together, each chamber (32, 34) comprising a wall extending from an interior surface (52, 54) of the base (12) of the chamber support (30) and the chamber wall having an interior surface (46, 48) and each chamber (32, 34) communicating with the mixing region (80) via a port (56, 58) in the base (12) of the chamber support (30);
c) piercing means (72, 74) which is arranged so that when it is desired to release a component from a receptacle (100) in the device the piercing means (72, 74) may be caused to pierce the receptacle (100);
d) plunger means (60) slidably engaged with the interior wall surface (46, 48) of each chamber (32, 34) suitable for expelling a component from the chamber (32, 34) into the mixing region (30);
e) a perforate mixing element (82) located in the mixing region (30);
f) means (94) for causing movement of the mixing element (82) in the mixing region (30) for mixing the components of the composition, and
g) a passageway (96) located between the chambers (32, 34) and communicating with the mixing region (30) at one end and providing a nozzle (98) at its other end so that the mixed composition may be expelled from the mixing region (30) via the passageway (96) upon bringing about relative movement of approach between the mixing housing (10) and the chamber support (30).

4. A device according to Claim 3 wherein the means for piercing (72, 74) a receptacle (100) is arranged so that the part of the receptacle (100) which is cut remains attached to the receptacle (100).

**5.** A device according to Claim 1 comprising

a) a mixing housing (210) comprising a base (212) having an interior surface (214) and a wall (216) having an interior wall surface (218);
b) a chamber support (230) having parallel chambers (232, 234), one for each component of the multi-component composition, the chamber support (230) being fixed to the wall (216) of the mixing housing (210), such that together they define a mixing region (280) into which the components of a multi-component composition may be introduced and mixed together, each chamber (232, 234) communicating with the mixing region (280) via a port (256, 258) in the chamber support (230);
c) a perforate mixing element (282) located in the mixing region (280);
d) piercing means (272, 274) which is arranged so that when it is desired to release a component from a chamber (232, 234) the piercing means (272, 274) may be caused to pierce a retaining surface to release the component from the chamber (232, 234);
e) plunger means (260) slidably engaged with the interior wall surface of each chamber (232, 234) suitable for expelling a component from the chamber (232, 234) into the mixing region (280);
f) a passageway (296) communicating with the mixing region (280) at one end and providing a nozzle (298) at its other end so that the mixed composition may be expelled from the mixing region (280) via the passageway (296) upon bringing about relative movement of approach between the mixing element (282) and the chamber support, and
g) means for causing movement of the mixing element (282) in the mixing region (280) for mixing the components of the composition.

**6.** The use of a device according to Claim 1 to form medical dressings.

## Patentansprüche

**1.** In einer Hand eines Benutzers zu haltende Vorrichtung zum manuellen Mischen und Austragen einer Siliconkomposition bestehend aus zwei oder mehreren für sich allein stabilen in getrennten Behältern (100) befindlichen Komponenten von im wesentlichen gleichem Volumen und Viskosität, mit einem einen die zu mischenden Komponenten aufnehmenden Mischbereich (80; 280) aufweisenden Mischgehäuse (10; 210), mit voneinander getrennten Aufnahmen für die Behälter (100) bildenden Kammern (32, 34; 232, 234), mit einer aus aufbrechbarem Material bestehenden Abdeckung, wobei die Kammern (32, 34; 232, 234) mit dem Mischbereich (80, 280) kommunizieren, mit Perforierungsmitteln (72, 74; 272 274), deren Anordnung ein Perforieren der Behälter (100) ermöglicht, wenn die Komponenten aus den Behältern (100) auszutragen sind, mit Kolbenelementen (60; 260) für das Austreiben der Komponenten aus den Kammern (32, 34; 232, 234) in den Mischbereich (80; 280) für das Mischen der Komponenten der Komposition, mit einem in dem Mischbereich (80; 280) angeordneten Mischelement (82; 282) und mit einem Durchgang (96; 296), in dem sich Mittel für das Betätigen des Mischelements (82; 282) befinden und der für den Austritt der gemischten Komposition aus dem Mischbereich (80; 280) dient, dies alles in derartiger Anordnung, daß für das Mischen und Austreiben der Komposition eine relative Annäherungsbewegung zwischen den Perforierungsmitteln (72, 74; 272, 274) und den Behältern (100) erzeugbar ist, die nach Aufbrechen der Behälter (100) als relative Annäherungsbewegung zwischen den Kolbenelementen (60; 260) und dem Mischbereich (10; 210) wirksam wird, um die Komponenten in den Mischbereich (80; 280) zu überführen, worauf das Mischelement (82; 282) zum Mischen der Komponenten betätigbar ist, und daß über die relative Annäherungsbewegung der Elemente der Vorrichtung die Komposition über den Durchgang (96; 296) aus der Vorrichtung austragbar ist.

**2.** Vorrichtung nach Anspruch 1, **gekennzeichnet durch** einen Kammerträger (30; 230) mit zwei zylindrischen Kammern (32, 34; 232, 234), deren Achsen parallel und zu beiden Selten der Mittellinie des Kammerträgers angeordnet sind, und durch zwei Kolben (62, 64; 262, 264) als Kolbenelemente (60; 260), die zwecks gleichzeitigem Verschieben in den Kammern (32, 34; 232, 234) starr miteinander verbunden sind.

**3.** Vorrichtung nach Anspruch 1, **gekennzeichnet durch**

a) ein Mischgehäuse (10) mit einer eine innere Oberfläche (14) und eine innere Wandung (16) aufweisenden Basis (12),
b) ein Kammerträger (30) mit parallel angeordneten Kammern (32, 34) zwecks Aufnahme je einer Komponente einer Mehrfachkomponenten-Komposition, wobei der Kammerträger (30) eine Basis (30) und eine äußere Oberfläche (38, 42) aufweisende Wandung (40) umfaßt und verschiebbar im Mischgehäuse (10) angeordnet ist, und die äußere Oberfläche (38) der Basis (30) des Kammerträgers (30) und die innere Oberfläche (14, 18) der Basis (12) sowie die Wandung (16) einen Mischbereich (80) begrenzen, in dem die Komponenten der Mehrfachkomponenten-Komposition überführt und gemischt werden, und wobei jede Kammer (32, 34) mit einer von der inneren Oberfläche (52, 54) der Basis (12) des Kammerträgers (30) sich erstreckenden Wandung kommuniziert,

c) Perforierungsmittel (72, 74) zum Perforieren der Behälter (100), deren Anordnung derart gewählt ist, daß nach Erfordernis zwecks Austrags der Komponenten aus den Behältern (100) diese perforierbar sind,

d) Kolbenelemente (60) für den verschiebbaren Eingriff mit den inneren Wandoberflachen (46, 48) einer jeden Kammer (32, 34) zwecks Austrag der Komponenten aus den Kammern in den Mischbereich (80),

e) ein in dem Mischbereich (80) angeordnetes, perforiertes Mischelement (82),

f) Mittel (24) für das Bewegen des Mischelements (82) zum Mischen der Komponenten der Komposition und

g) einen zwischen den Kammern (32, 34) befindlichen, mit dem einen Ende des Mischbereichs (80) kommunizierenden Durchgang (96), der an seinem freien Ende eine Düse (98) bildet, dies alles in derartiger Anordnung, daß die Komposition über den Durchgang aus dem Mischbereich (80) ausgetragen werden kann, sobald eine relative Annäherungsbewegung zwischen dem Mischgehäuse (10) und dem Kammerträger (30) aufgebracht wird.

4. Vorrichtung nach Anspruch 3, **dadurch gekennzeichnet, daß** die Mittel für das Perforieren (72, 74) der Behälter (100) derart ausgebildet und angeordnet sind, daß das perforierte Stück des Behälters (100) an diesem verbleibt.

5. Vorrichtung nach Anspruch 1, **gekennzeichnet durch**

a) ein Mischgehäuse (210) mit einer eine innere Oberfläche (240) und eine innere Wandung (218) aufweisenden Basis (212),

b) ein Kammerträger (230) mit parallel angeordneten Kammern (232, 234) zwecks Aufnahme je einer Komponente einer Mehrkomponenten-Komposition, wobei der Kammerträger (230) an der Wandung (260) des Mischgehäuses (210) zwecks Bildung eines Mischbereichs (280) befestigt ist, in den die Komponenten der Komposition eingeführt und gemischt werden und der mit jeder der Kammern (232, 234) über eine Öffnung (256, 258) in dem Kammerträger (230) kommuniziert,

c) ein perforiertes, im Mischbereich (280) angeordnetes Mischelement (282),

d) Perforierungsmittel (272, 274), deren Anordnung so gewählt ist, daß nach Erfordernis zwecks Austrag der Komponenten aus den Kammern (232, 234) aus diesen eine Rückhaltefläche perforierbar ist,

e) ein Kolbenelement (260) für den verschiebbaren Eingriff mit den inneren Wandungsoberflächen einer jeden Kammer (232, 234) zwecks Austrag der Komponenten aus den Kammern (232, 234) in den Mischbereich (280),

f) einen mit einem Ende mit dem Mischbereich kommunizierender und am anderen Ende eine Düse (298) bildender Durchgang (296) zwecks Austrags der gemischten Komposition aus dem Mischbereich (280) sobald eine relative Annäherungsbewegung zwischen dem Mischelement (282) und dem Kammerträger aufgebracht wird und

g) Mittel für das Bewegen des Mischelements (282) in dem Mischbereich (280) zwecks Mischen der Komponenten der Komposition.

6. Vorrichtung nach Anspruch 1, **gekennzeichnet durch** ihre Verwendung zur Erzeugung von medizinischen Beschichtungen.

## Revendications

1. Un dispositif de mélange et de distribution conçu pour être tenu dans une main d'un opérateur pour mélanger ensemble les composants d'une composition à base de silicone présentés sous la forme de deux, ou plus, composants stables individuels de viscosité et de volume sensiblement égaux contenus chacun dans un récipient (100), et pour distribuer la composition après mélange par une opération manuelle, le dispositif comprenant un boîtier de mélange (10; 210) comportant une région de mélange (80; 280) dans laquelle les composants de la composition à plusieurs composants peuvent être introduits et mélangés ensemble, les chambres séparées (32, 34; 232, 234) comprenant chacune un desdits récipients (100), ou formant un récipient (100) comportant une fermeture réalisée en un matériau qui peut être rompu, chaque chambre (32, 34; 232, 234) communiquant avec la région de mélange (80; 280), des moyens de perçage (72, 74; 272, 274) agencés de manière que lorsqu'on désire faire sortir un composant de son récipient (100), les moyens de perçage (72, 74; 272, 274) peuvent provoquer le perçage du récipient (100), un moyen plongeur (60; 260) pour pousser un composant hors de la chambre (32, 34; 232, 234) par forçage de celui-ci dans la région de mélange (80; 280), un organe mélangeur (82; 282) disposé dans la région de mélange (80; 280) pour mélanger les composants de la composition, et un passage (96; 296) qui contient un moyen pour actionner l'organe mélangeur (82; 282) et à travers lequel la composition après mélange peut être expulsée de la région de mélange (80; 280), la structure et l'agencement étant tels que lorsque l'on souhaite mélanger et distribuer la composition, on peut provoquer un mouvement relatif d'approche entre les moyens de perçage (72, 74; 272, 274) et les récipients (100) pour provoquer la rupture des récipients (100), on peut provoquer un mouvement relatif d'approche entre le moyen plongeur (60; 260) et le boîtier de mélange (10; 210) pour pousser le composant dans la région de mélange (80; 280), on peut actionner l'organe de mélange (82; 282) pour mélanger les composants dans la région de mélange (80; 280), et on peut provoquer un mouvement relatif d'approche entre les organes du dispositif pour faire sortir la composition après mélange hors du dispositif, par ledit passage (96; 296).

2. Un dispositif conforme à la revendication 1, comprenant un support de chambre (30; 230) qui comprend deux chambres cylindriques (32, 34; 232, 234) dont les axes sont parallèles et disposés de chaque côté de, et parallèlement à, une ligne centrale du support de chambre, et le moyen plongeur (60; 260) comprenant deux plongeurs (62, 64; 262, 264) qui sont rigidement liés l'un à l'autre et qui sont montés pour glisser simultanément dans les chambres (32, 34; 232, 234).

3. Un dispositif conforme à la revendication 1, comprenant,

a) une chambre de mélange (10) comprenant une base (12) présentant une surface intérieure (14) et une paroi (16) présentant une surface de paroi intérieure (18);
b) un support de chambre (30) définissant deux chambres parallèles (32, 34) une pour chacun des composants d'une composition à plusieurs composants, le support de chambre (30) comprenant une base (36) et une paroi (40), chacune présentant une surface extérieure (38, 42), le support de chambre (30) étant coulissant dans le boîtier (10) et la surface extérieure (38) de la base (30) du support de chambre et les surfaces intérieures (14, 18) de la base de boîtier (12) et de la paroi de boîtier (16) étant telles qu'elles définissent ensemble une région de mélange (80) dans laquelle on peut introduire les composants de la composition à plusieurs composants et les mélanger, chaque chambre (32, 34) comprenant une paroi s'étendant d'une surface intérieure (52, 54) de la base (12) du support de chambre (30) et la paroi de chambre présentant une surface intérieure de chambre (46, 48) et chaque chambre (32, 34) communiquant avec la région de mélange (80) par l'intermédiaire d'un trou (56, 58) formé dans la base (12) du support de chambre (30);
c) des moyens de perçage (72, 74) qui sont agencés de manière que, lorsqu'on souhaite faire sortir un composant d'un récipient (100) dans le dispositif, on peut provoquer le perçage du récipient par lesdits moyens de perçage (72, 74);
d) un moyen plongeur (60) coulissant contre la surface de paroi intérieure (46, 48) de chaque chambre (32, 34), convenant pour faire sortir un composant de la chambre (32, 34) et le faire entrer dans la région de mélange (30);
e) un organe de mélange perforé (82) disposé dans la région de mélange (30);
f) un moyen (94) pour provoquer un mouvement de l'organe de mélange (82) dans la région de mélange (30) de manière à mélanger les composants de la composition, et
g) un passage (96) disposé entre les chambres (32, 34) et communiquant avec la région de mélange (30) à une extrémité et constituant un ajutage (98) à son autre extrémité de manière que la composition après mélange puisse être expulsée de la région de mélange (30) par l'intermédiaire du passage (96) après que l'on ait provoqué un mouvement relatif d'approche entre le boîtier de mélange (10) et le support de chambre (30).

4. Un dispositif conforme à la revendication 3, dans lequel les moyens de perçage (72, 74) d'un récipient (100) sont agencés de manière que la partie du récipient (100) qui est perforée reste attachée au réceptacle (100).

5. Un dispositif conforme à la revendication 1, comprenant

a) un boîtier de mélange (210) comprenant une base (212) présentant une surface intérieure (214) et une paroi (216) présentant une surface de paroi intérieure (218);
b) une chambre de support (230) présentant des chambres parallèles (232, 234), pour chacun des composants de la composition à composants multiples, le support de chambre (230) étant fixé à la paroi (216) du boîtier de mélange (210), de manière à définir ensemble une région de mélange (280) dans laquelle les composants d'une composition à plusieurs composants peuvent être introduits et mélangés ensemble, chaque chambre (232, 234) communiquant avec la région de mélange (280) par l'intermédiaire d'un trou (256, 258) formé dans un support de chambre (230);
c) un organe de mélange perforé (282) disposé dans la région de mélange (280);
d) des moyens de perçage (272, 274) agencés de manière que lorsque l'on souhaite faire sortir un composant d'une chambre (232, 234), on peut provoquer le perçage d'une surface de retenue par les moyens de perçage (272, 274) de manière à libérer le composant de la chambre (232, 234);

e) un moyen plongeur (260) coulissant contre la surface de paroi intérieure de chaque chambre (232, 234) et convenant pour faire sortir un composant de la chambre (232, 234) et le faire entrer dans la région de mélange (280);

f) un passage (296) communiquant avec la région de mélange (280) à une extrémité et constituant un ajutage (298) à son autre extrémité de manière que la composition après mélange puisse être expulsée de la région de mélange (280) à travers le passage (296) par un mouvement relatif d'approche entre l'organe de mélange (282) et le support de chambre, et

g) un moyen pour provoquer un mouvement de l'organe de mélange (282) dans la région de mélange (280) pour mélanger les composants de la composition.

6. Utilisation du dispositif conforme à la revendication 1, pour la réalisation de pansements médicaux.

FIG.:1

FIG.:2

FIG.:3

FIG.:4

FIG.:5

FIG.:6

FIG.:7

FIG.:9

FIG.:8

FIG.: 12

FIG.:10

FIG.:11